# EUROPEAN PATENT SPECIFICATION

(11) **EP 3 954 384 B1**
(45) Date of publication and mention of the grant of the patent: **02.07.2025**
(21) Application number: 21195581.0
(22) Date of filing: 10.09.2018
(51) Int. Cl.: A61K 39/00, A61K 33/08, A61P 3/10, A61P 37/02, G01N 33/564, C12Q 1/6883

(54) **GENOTYPE STRATIFICATION IN DIABETES TREATMENT AND PREVENTION**
GENOTYPSTRATIFIZIERUNG BEI DER BEHANDLUNG UND VORBEUGUNG VON DIABETES
STRATIFICATION DU GÉNOTYPE DANS LE TRAITEMENT ET LA PRÉVENTION DU DIABÈTE

(30) Priority: 08.09.2017 SE 1751094
(43) Date of publication of application: 16.02.2022
(62) Divisional of application: 18854165.0
(73) Proprietor: Diamyd Medical AB, 111 56 Stockholm (SE)
(72) Inventor: ESSEN-MÖLLER, Anders, 115 25 Stockholm (SE)
(74) Representative: Zacco Sweden AB

(56) References cited:
- ERLICH H. ET AL: "HLA DR-DQ Haplotypes and Genotypes and Type 1 Diabetes Risk: Analysis of the Type 1 Diabetes Genetics Consortium Families", vol. 57, no. 4, 1 April 2008 (2008-04-01), US, pages 1084 - 1092, XP055873928, ISSN: 0012-1797, Retrieved from the Internet <URL:https://diabetes.diabetesjournals.org/content/diabetes/57/4/1084.full.pdf> DOI: 10.2337/db07-1331
- BONIFACIO EZIO ET AL: "Effects of High-Dose Oral Insulin on Immune Responses in Children at High Risk for Type 1 Diabetes : The Pre-POINT Randomized Clinical Trial", vol. 313, no. 15, 21 April 2015 (2015-04-21), US, pages 1541 - 1549, XP055874125, ISSN: 0098-7484, Retrieved from the Internet <URL:http://dx.doi.org/10.1001/jama.2015.2928> DOI: 10.1001/jama.2015.2928
- EZIO BONIFACIO ET AL: "Supplement 1: Pre-POINT STUDY PROTOCOL Pre-POINT (Primary Oral INsulin Trial) (Effects of High-Dose Oral Insulin on Immune Responses in Children at High Risk for Type 1 Diabetes : The Pre-POINT Randomized Clinical Trial)", JAMA THE JOURNAL OF THE AMERICAN MEDICAL ASSOCIATION, vol. 313, no. 15, 21 April 2015 (2015-04-21), US, pages 1 - 55, XP055874154, ISSN: 0098-7484, Retrieved from the Internet <URL:https://cdn.jamanetwork.com/ama/content_public/journal/jama/933762/jpc150002supp1_prod.pdf?Expires=1642699285&Signature=Atpcu1dHeGPQMG1m8LxwqRU8MSEQUIOTFUxQCfIaty~MBMgPusMVaI9~f4QaXHcTk0dBUHNyAeVMWr1a5HK4oxOAaSq9pEyZxCtCd5swPMDQopon0XPuweM2p0P9QhJF0lomRCMzqh3K2CvJtygBq5nx7-HjJq7m4oCkZRv5Fzry0tZo2Qls> DOI: 10.1001/jama.2015.2928
- BONIFACIO EZIO ET AL: "Supplementary Online Content: Effects of High-Dose Oral Insulin on Immune Responses in Children at High Risk for Type 1 Diabetes : The Pre-POINT Randomized Clinical Trial", JAMA THE JOURNAL OF THE AMERICAN MEDICAL ASSOCIATION, vol. 313, no. 15, 21 April 2015 (2015-04-21), US, pages 1 - 13, XP055874153, ISSN: 0098-7484, Retrieved from the Internet <URL:https://cdn.jamanetwork.com/ama/content_public/journal/jama/933762/jpc150002supp2_prod.pdf?Expires=1642699285&Signature=bnVkzdGoQVvmrYHd29J~ALlPCv5SOLFcTLhtRMgY8SaZ3eHHG8J~at~DJNuw-K2r72JoWjdVmQWjDiPYYsNliPh7yG4D9NkVqplgfYm-pNiDUQQEGtZ1nRJUQExZ~fcfOIkEqS1gcOWRv7idkxZnddw~t5I1p53Rx3JrMOQfssPtoM0lIGJC> DOI: 10.1001/jama.2015.2928
- NANTO-SALONEN K ET AL: "Nasal insulin to prevent type 1 diabetes in children with HLA genotypes and autoantibodies conferring increased risk of disease: a double-blind, randomised controlled trial", THE LANCET, ELSEVIER, AMSTERDAM, NL, vol. 372, no. 9651, 15 November 2008 (2008-11-15), pages 1746 - 1755, XP025658173, ISSN: 0140-6736, [retrieved on 20081113], DOI: 10.1016/S0140-6736(08)61309-4
- JOHNNY LUDVIGSSON ET AL: "GAD65 antigen therapy in recently diagnosed type 1 diabetes mellitus", THE NEW ENGLAND JOURNAL OF MEDICINE, vol. 366, no. 5, 2 February 2012 (2012-02-02), pages 433 - 442, XP055434070, DOI: 10.1056/NEJMoa1107096
- LUDVIGSSON JOHNNY ET AL: "GAD-treatment of children and adolescents with recent-onset type 1 diabetes preserves residual insulin secretion after 30?months : The GAD-Alum Phase II and III Trials", DIABETES/METABOLISM RESEARCH AND REVIEWS, vol. 30, no. 5, 1 July 2014 (2014-07-01), GB, pages 405 - 414, XP055796913, ISSN: 1520-7552, DOI: 10.1002/dmrr.2503
- HANNELIUS ULF ET AL: "Efficacy of GAD-alum immunotherapy associated within recently diagnosed type 1 diabetes", DIABETOLOGIA, SPRINGER, BERLIN, DE, vol. 63, no. 10, 5 August 2020 (2020-08-05), pages 2177 - 2181, XP037238798, ISSN: 0012-186X, [retrieved on 20200805], DOI: 10.1007/S00125-020-05227-Z
- VAN RAMPELBERGH, J: "@ lmcyse Phase lb clinical trial of IMCY-0098 in young adults with recent-onset type 1 diabetes", 1 October 2019 (2019-10-01), pages 1 - 1, XP093028833, Retrieved from the Internet <URL:https://www.arianapharma.com/wp-content/uploads/2019/10/Poster-T1D-EASD-Final-PDF.pdf> [retrieved on 20230303]
- WEN LI ET AL: "Human DQ8 Can Substitute for Murine I-Ag7 in the Selection of Diabetogenic T Cells Restricted to I-Ag71", THE JOURNAL OF IMMUNOLOGY, vol. 168, no. 7, 1 April 2002 (2002-04-01), US, pages 3635 - 3640, XP093109976, ISSN: 0022-1767, Retrieved from the Internet <URL:https://journals.aai.org/jimmunol/article/168/7/3635/34861/Human-DQ8-Can-Substitute-for-Murine-I-Ag7-in-the> DOI: 10.4049/jimmunol.168.7.3635
- LEE KON HO ET AL: "Structure of a human insulin peptide-H LA-DQ8 complex and susceptibility to type 1 diabetes", NAT IMMUNOL, vol. 2, 1 June 2001 (2001-06-01), United States, pages 501 - 507, XP093110026, Retrieved from the Internet <URL:https://www.nature.com/articles/ni0601_501>
- TIAN J ET AL: "NASAL ADMINISTRATION OF GLUTAMATE DECARBOXYLASE (GAD65) PEPTIDES INDUCES TH2 RESPONSES AND PREVENTS MURINE INSULIN-DEPENDENT DIABETES", JOURNAL OF EXPERIMENTAL MEDICINE, ROCKEFELLER UNIVERSITY PRESS, US, vol. 183, no. 4, 1 January 1996 (1996-01-01), pages 1561 - 1567, XP009032068, ISSN: 0022-1007, DOI: 10.1084/JEM.183.4.1561
- ASPORD C ET AL: "Nasal administration of CTB-insulin induces active tolerance against autoimmune diabetes in non-obese diabetic (NOD) mice", CLINICAL AND EXPERIMENTAL IMMUNOLOGY, WILEY-BLACKWELL PUBLISHING LTD, GB, vol. 130, no. 2, 17 October 2002 (2002-10-17), pages 204 - 211, XP071079582, ISSN: 0009-9104, DOI: 10.1046/J.1365-2249.2002.01988.X
- LIU YUK-FUN ET AL: "Immune and Metabolic Effects of Antigen-Specific Immunotherapy Using Multiple [beta]-Cell Peptides in Type 1 Diabetes", DIABETES, vol. 71, no. 4, 1 April 2022 (2022-04-01), US, pages 722 - 732, XP093109975, ISSN: 0012-1797, Retrieved from the Internet <URL:https://diabetesjournals.org/diabetes/article/71/4/722/140961/Immune-and-Metabolic-Effects-of-Antigen-Specific> DOI: 10.2337/db21-0728
- ZHAO LUE PING ET AL: "Oral Insulin Delay of Stage 3 Type 1 Diabetes Revisited in HLA DR4-DQ8 Participants in the TrialNet Oral Insulin Prevention Trial (TN07)", DIABETES CARE, vol. 47, no. 9, 1 July 2024 (2024-07-01), US, pages 1608 - 1616, XP093234261, ISSN: 0149-5992, Retrieved from the Internet <URL:https://diabetesjournals.org/care/article-pdf/47/9/1608/780314/dc240573.pdf> DOI: 10.2337/dc24-0573

## Description

### Technical field

The present invention relates to the technical field of medicinal treatment, and in particular to methods for immunotherapeutic treatment of patients based on said patients' genetic profile, as well as compounds and compositions for use in such methods.

### Background art

Type 1 diabetes mellitus (T1DM) is an autoimmune disease characterized by an immune-mediated destruction of the insulin-secreting cells, the beta-cells, of the pancreas. T1DM often has an early onset, already in childhood.

The production of autoantibodies to the beta cells causes a degradation of the beta cells. The degradation process occurs over many years and eventually results in metabolic abnormalities. These abnormalities are first manifested as impaired glucose tolerance and progress to symptomatic hyperglycemia. The antibodies that have been identified in association with the development of T1DM are antibodies to insulin (IAA), GAD65 (GADA including truncated GADA or tGADA), IA-2 (IA-2A) and ZnT8 (ZnT8A).

It has been suggested that administration of alum-formulated glutamic acid decarboxylase can preserve beta-cell function in patients with recent-onset T1DM (Ludvigsson et al., N Engl J Med. 2012 Feb 2;366(5):433-42).

Also insulin has been used as an antigen component in immunotherapy for T1DM (Ali et al, Sci Transl Med. 2017 Aug 9;9(402)).

The 6 year incidence of diabetes-associated autoantibodies in genetically at-risk children has been described by Krischer et al. (Diabetologia. 2015 May;58(5):980-7).

Immunomodulation by a therapeutic medication intended for treatment of diabetes and prevention of autoimmune diabetes is known in the art, *i.a*. from EP1755631 and EP3151853.

Bonifacio E. et al. JAMA (2015) 313(15):1541-1549 and Nanto-Salonen K. et al. Lancet (2008) 372(9651):1746-1755 teach studies analysing immunological and therapeutic effects of oral insuline administration in at risk of type I diabetes.

### Summary of the invention

The number and type of antibodies are predictive of progression to diabetes.

Patients with different genotypes, have different sets and development of antibodies leading to degradation of the beta cells. This in turn will lead to a difference in progression of the disease, based on which antibody is triggered in the patient.

By identifying the genotype and measuring and quantifying the amount of antibodies against GAD65 and insulin simultaneously, it is possible to customize a vaccine for prevention or treatment of T1DM.

The invention is defined by the appendant claims.

In one aspect, the disclosure which is not part of the invention relates to a method for treatment or prevention of an autoimmune disease in an individual, comprising determining the HLA haplotype of the individual; and subjecting the individual to a treatment regimen based on said haplotype.

In one embodiment, the method comprises determining the HLA haplotype of the individual, determining the specificity of the initially occurring autoantibody related to the autoimmune disease in the individual; and subjecting the individual to a treatment regimen based on said haplotype and specificity of said initially occurring autoantibody.

In one embodiment the disease is autoimmune diabetes, such as Type 1 Diabetes Mellitus.

In one embodiment an individual having an HLA DR3-DQ2 haplotype, and optionally presenting with GADA first, is subjected to treatment with at least a GAD-autoantigen.

In one embodiment an individual having an HLA DR3/4-DQ2/8 genotype is subjected to treatment with both a GAD autoantigen and an insulin autoantigen in the same or separate formulations.

In one embodiment an individual having a DR4-DQ8 haplotype but not a DR3-DQ2 haplotype, and optionally presenting with insulin autoantibodies first, is subjected to treatment with at least an insulin autoantigen.

In one embodiment an individual having a DR4-DQ8 but not a DR3-DQ2 haplotype is subjected to treatment with alum alone.

In one embodiment an individual having an HLA DR8/4-DQ4/8 haplogenotype, and optionally presenting with insulin autoantibodies first, is subjected to treatment with at least an insulin autoantigen.

In one embodiment an individual having a DQ2 genotype and presenting with GAD antibodies first is subjected to treatment with a GAD antigen.

In one embodiment an individual having a HLA-DR4/4-DQ8/8 genotype is subjected to treatment with an insulin antigen.

In one embodiment an individual having a HLA-DR8/4-DQ4/8 genotype is subjected to treatment with an insulin antigen.

In one embodiment an individual having a HLA-DR3/3-DQ2/2 genotype is subjected to treatment with a GAD antigen.

In one embodiment an individual having a HLA- DR3/4-DQ2/8 genotype is subjected to treatment with a GAD antigen.

In one embodiment an administration of GAD-antigen is one of subcutaneous, intradermal, or intra-lymphatic.

In one embodiment a GAD antigen is formulated with alum.

In one embodiment an administration of insulin autoantigen is oral, sublingual, intramuscular, intradermal, or intra-lymphatic.

In one embodiment an insulin antigen is formulated with alum or in saline solution.

In one embodiment an administration of alum alone is subcutaneous, intradermal, or intra-lymphatic.

In one aspect, the disclosure which is not part of the invention relates to GAD for use as an autoantigen in a method according to the above.

The invention relates to insulin for use as an autoantigen in a method according to the above.

In one aspect, the disclosure which is not part of the invention relates to the use of GAD in the manufacture of a pharmaceutical composition for use in a method according to the above.

In one aspect, the disclosure which is not part of the invention relates to the use of insulin in the manufacture of a pharmaceutical composition for use in a method according to the above.

### Definitions

All terms as used herein are intended to have the meaning given to them by the person of ordinary skill in the art in the context of the disclosure. A few terms are specifically defined below in order to avoid ambiguity.

The term "alum" refers to aluminium hydroxide which is commonly used as an adjuvant in pharmaceutical compositions for use in immunotherapy. "Alum alone" refers to a composition comprising alum but no antigen.

The term "GAD" refers to the protein glutamic acid decarboxylase and includes isoforms of GAD such as GAD38, GAD65 and GAD67, as well as fragments thereof.

The term "insulin", when used for insulin as an autoantigen, shall be construed as including preproinsulin, proinsulin, as well as fragments thereof.

The term T1D includes all types of diabetes where autoantibodies to beta-cell autoantigens are present such as Type 1 Diabetes Mellitus (T1DM), 1,5 diabetes, LADA, LADY, SPIDDM, PIDM and more.

The terms "a" and "an" shall be construed as including both the singular and the plural.

### Detailed description

The etiology of T1DM has yet to be clarified. However the pathogenesis is marked by autoantibodies against insulin (IAA), GAD65 (GADA), IA-2 (IA-2A) or ZnT8 (ZnT8A).

Recent investigations have found that two major groups of children developing islet autoantibodies exist; one that present with IAA as a first autoantibody and this group often include children of low age with HLA genotypes including the DR4-DQ8 haplotype, and the other group often somewhat older children that present with GADA or tGADA first, often of DR3-DQ2-haplotype 2, leading the inventors of the present invention to test the efficacy of GAD-alum versus placebo in these HLA-groups (cf. Example 2).

The HLA (Human Leukocyte Antigen) gene family provides for a group of proteins known as HLA complex. The HLA complex helps the immune system to distinguish self from non-self. Within the HLA complex there are three basic groups, MHC (major histocompatibility complex) class I, MHC class II and MHC class III. The MHC class II genes comprise *HLA-D* genes.

A TEDDY (The Environmental Determinants of Diabetes in the Young) substudy has been performed aimed at identifying genetic and environmental factors that explains the trigger of the first islet autoantibody.

In the study the development of islet autoimmunity and T1DM was studied. Enrolled into the study were children - from newborn infants up to children of age 15 years, with a genetic susceptibility for T1DM.

Participants were submitted to clinical visits every 3 months. At each visit blood was analyzed with regards to GADA, IAA, IA-2A, ZnT8A, DNA, mRNA, infectious agents, HbA1c, PBMC, erythrocytes, storage plasma/serum. Analysis was also performed on urinary samples, nasal swabs, tap water, toenail clippings and salivary cortisol. Stool samples were collected monthly during the first 48 months, and thereafter quarterly.

In addition to the analysis mentioned above, interviews were performed regarding maternal pregnancy diet (FFQ of selected foods), smoking; negative life events, parental anxiety, depression, records of infections, medications, immunizations, family history, DNA from First Degree Relatives (FDRs), Physical activity assessment. There was also a re-enrolment of subjects lost.

The aim was to study prenatal factors that may affect the risk for HLA-dependent autoantibody appearance. Non-diabetic mothers of singleton infants (n=6,947) filled out a questionnaire 3-4.5 months after pregnancy. Lower birth weight was defined as the lower 25% of the birth weights of the TEDDY children.

Maternal factors such as smoking, BMI, drinking during the 3rd trimester (>2 drinks/month), and maternal infections (lower respiratory tract infection, skin infection or rash, genital infection). After adjusting for country, T1DM in a FDR, HLA genotypes and gender, neither of these maternal factors were associated with a first islet autoantibody.

Among FDR-children IAA-only as first autoantibody was more common than GADA-only at an early age, but not at a later age
Compared to children with genotype *HLA-DR3*/*4,* children with genotype *HLA-DR3*/*3* showed a reduced risk of manifesting IAA-only as the first islet autoantibody.

Among children with genotype *HLA-DR4*/*4* and *HLA-DR4*/*8,* there was a reduced risk for GADA-only as the first islet autoantibody.

Among girls there was a reduced risk of IAA-only as the first islet autoantibody, but not GADA-only as the first islet autoantibody.

Among children born with a lower birth weight, there was a reduced risk of GADA-only as the first islet autoantibody.

The appearance of IAA-only was more common in boys and associated with *HLA-DR4*/*4-DQ8*/*8* and *HLA-DR8*/*4-DQ4*/*8.*

The appearance of GADA-only was not associated with gender, but associated with *HLA-DR3*/*3-DQ2*/*2* and *HLA-DR3*/*4-DQ2*/*8,* and less common in children with low birth weight.

It could be concluded that GADA-only as the first islet autoantibody is triggered primarily in individuals carrying the *HLA-DQ2* haplotype. Maternal factors affecting birth weight may affect the appearance of GADA as the first autoantibody.

Individuals with genotypes *HLA-DR4*/*4-DQ8*/*8* and *HLA-DR8*/*4-DQ4*/*8* generally primarily show presence of IAA as first islet autoantibody.

Individuals with genotypes *HLA-DR3*/*3-DQ2*/*2* and *HLA-DR3*/*4-DQ2*/*8* generally primarily show presence of GADA as first islet antibody
Surprisingly the present inventors could show that administration of a GAD-autoantigen to T1DM patients that had an HLA-haplotype associated with GADA as the first autoantibody, favorably can be used in treatment/prevention regimens for T1DM. The antigen to be used may thus be decided based on the genotype of the patient.

In addition it could be shown that alum alone can delay the decline of stimulated C-peptide in patients carrying the DR4-DQ8, but not the DR3-DQ2 haplotype.

According to the present invention, an autoantigen can be administered by intralymphatic injection, injection directly into a lymph node, intradermal injection, subcutaneous injection, intramuscular injection, intraperitoneal injection, intravenous injection, subcutaneous injection, intranasal, transmucosal or sublingual application; or orally, including administration as tablets, pellets, granules, capsules, lozenges, aqueous or oily solutions, suspensions, emulsions, sprays or as reconstituted dry powdered form with a liquid medium.

In one embodiment of this invention administration of autoantigen is made directly into the lymph nodes or into the lymphatic system to allow resident APCs to present antigen peptides to the immune system. If administration of autoantigens is made directly into a lymph node or into the lymphatic system, the dose will preferably be between 1 and 15 µg per autoantigen, more preferred between 2 and 10 µg per autoantigen or 2 to 5 µg per autoantigen. Formulation in alum is preferred.

According to certain embodiments, the at least one autoantigen is administered intrainguinal, intralymph node or intralymphatic. In some embodiments, the volume for intra-inguinal injection of the antigens is between 0.05 and 0.2 ml, more preferred between 0.05 and 0.15 ml.

According to certain embodiments, where the at least one antigen is administered by intralymph-node or intralymphatic injection a preferred dosage is between 1-15ug, more preferred between 2-10, and most preferred between 2-5ug per injection and autoantigen used, such administrations taking place at least 2 times, more preferred at least 3 times and most preferred at least 4 times, at least 14 days apart, more preferably at least 30 days apart.

According to certain embodiments, at least one antigen and at least one IL-10 inducing compound are administered simultaneously. According to certain embodiments, the at least one antigen and the at least one IL-10 inducing compound are administered separately.

The method may further comprise a pretreatment of the individual to adjust the serum vitamin-D level, and such pretreatment may comprise administration of vitamin-D and/or vitamin-D analogs, and/or exposure to UVB-radiation, preferably for between 7 to 90 days before administration of the composition comprising at least one beta cell autoantigen to said subject, as described in EP3151853.

### Examples

The examples below are for reference purposes only.

### Example 1: TrialNet Phase II

Wherrett DK et al., (Lancet, 2011 Jul 23;378(9788):319-27) reported from a three arm (A: 3 sc x 20 µg GAD-alum (n=48); B: 2 x 20 µg GAD-alum and 1 of alum (n=49); and C: 3 x alum (n=48), randomized study in subjects diagnosed with T1D within 100 days and aged 3-45 years, that at 1 year, the 2-h AUC of C-peptide, adjusted for age, sex, and baseline C-peptide value, was A 0.412 nmol/L (0.349-0.478) in the GAD-alum group, B 0.382 nmol/L (0.322-0.446) in the GAD-alum plus alum group, and C 0.413 nmol/L (0.351-0.477) in the alum group corresponding to a loss in mean C-peptide at one year of 44%, 42% and 41% of baseline mean for GAD-alum x3, GAD-alum x2, and alum x3 respectively. The authors point out that the levels of C-peptide at baseline and at one year mirrored the findings in the control groups of three other TrialNet studies in subjects treated within 3 months of diagnosis, which also demonstrate that alum alone has no effect on the loss of insulin secretion at 12 months.

Adjusted declines over 12 months are thus 3,7%, 3,5%, and 3,42% monthly. Unadjusted values for up to 24 months are shown in the table below. Here the groups A, B and C showed a decline of and 58,3%, 62,3%, 55,1% over two years at or monthly 2,42%, 2,6%, 2,3%. Alum being marginally best.

**Table 1: Mean AUC C-peptide over 24 months**

| Time | Al*3 | | GAD-Al*2 | | GAD-Al*3 | |
|---|---|---|---|---|---|---|
| | Mean time* (mths) | Mean C-peptide | Mean time* (mths) | Mean C-peptide | Mean time* (mths) | Mean C-peptide |
| Baseline | -0.83 | 0.697 | -0.736 | 0.660 | -0.75 | 0.736 |
| 3 mths | 2.87 | 0.637 | 2.88 | 0.561 | 2.90 | 0.683 |
| 6 mths | 6.12 | 0.537 | 6.03 | 0.530 | 5.98 | 0.545 |
| 9 mths | 9.07 | 0.477 | 9.07 | 0.431 | 9.00 | 0.521 |
| 12 mths | 12.0 | 0.418 | 12.0 | 0.350 | 12.0 | 0.448 |
| 18 mths | 18.0 | 0.365 | 18.1 | 0.284 | 18.0 | 0.334 |
| 24 mths | 24.1 | 0.313 | 24.0 | 0.249 | 23.8 | 0.307 |

However, hypothesizing that GAD as an immunomodulating antigen is efficient in the HLA-groups that usually present with GAD-antibodies first, and that the Insulin-family of antigens is efficient in haplotypes that often present with Insulin antibodies first, the data from the study has now been revisited by the present inventors and it was surprisingly found that GAD-alum was almost twice as effective as alum alone (2,35% versus 4,45% change/month), in the DR3-DQ2 haplotype group that normally present with GAD antibodies first. In addition GAD-alum's efficiency in the groups that include DR4-DQ8 which usually present with IAA first (although DR3/DR4-DQ2/DQ8's may present with either GADA or IAA first) was only at a 40% advantage. And of particular interest was that the adjuvant alum (placebo) alone was more effective than the active drug in the DR4-DQ8 haplotype group, which is reported to include subjects that often present with IAA first.

**Table 2: Change in stimulated C-peptide at 1 year related to haplotype.**

| ***Number of pats & DQ*** | ***Arm*** | ***Ab*** | ***Change Stim C-pep AUC at 1 yr (%)*** | ***Change Stim C-pep AUC per month (%)*** |
|---|---|---|---|---|
| 7 with DQ2 | 3 injections 20ug GAD-alum | GADA | -22,03 | -1,84 |
| 7 with DQ2 | 2 injections 20ug GAD-alum plus by one alum alone | GADA | -34,33 | -2,86 |
| *14 with DQ2* | *Average active arms* | *GADA* | *-28,18* | *-2,35* |
| 4 with DQ2 | 3 injections alum only | GADA | -53,37 | -4,45 |

| ***Number of pats & DQ*** | ***Arm*** | ***Ab*** | ***Change Stim C-pep AUC at 1 yr (%)*** | ***Change Stim C-pep AUC per month (%)*** |
|---|---|---|---|---|
| 19 with DQ8 | 3 injections 20ug GAD-alum | IAA | -42,84 | *-*3,57 |
| 13 with DQ8 | 2 injections of 20ug GAD-alum plus one alum alone | IAA | -37,21 | -3,10 |
| *32 with DQ8* | *Average active arms* | *IAA* | *-40,55* | *-3,38* |
| 16 with DQ8 | 3 injections alum only | IAA | -22,48 | -1,87 |

### Example2: DiaPrevit

A randomized double-blind, placebo-controlled study in 50 healthy children with multiple islet autoantibodies but not yet with insulin requiring diabetes (DiaPREV-IT, ClinicalTrials.gov Identifier: NCT01122446), found that preventive treatment with GAD-alum did not affect progression to onset of clinical T1D.

The study was conducted in children aged 4-17.9 years (median age 5.2) with GADA and at least one additional islet autoantibody. Enrollment was completed in 2012 and the follow up period was 5 years. Eligible children, from the Diabetes Prediction in Skåne (DiPiS), The Environmental Determinants of Diabetes in the Young (TEDDY) studies, received two subcutaneous injections of 20 ug GAD-alum(n=25) or placebo (n=25), 30 days apart. Among the exclusion criteria was positivity for HLA DQB1*06:02.

Islet cell autoantibodies predict clinical onset of T1D and a child with more than one islet autoantibody runs a 70 % risk to develop T1D within 10 years. The sample size of 50 children randomized 1:1 to treatment with either GAD-alum (active) or alum alone (placebo), was based upon the assumption that 50 % of untreated children with more than one autoantibody will present with clinical T1D within a 5-year period. Surprisingly however, it was found that, although half of the children had impaired glucose metabolism at baseline, indicating a very high risk of progression to clinical disease, the individuals presenting with clinical T1D during the 5-year follow-up period were only 18 out of 50 (36%) and not 50 % as expected. In light of the reported overall non-significant results, an investigation was performed to evaluate which, if any, subgroups could explain the lower than expected incidence of overt T1D cases being presented.

It was found that 12 of the 18/50 children who progressed to T1D within the 5-year follow-up period (170-1830 days after the first injection of study drug) were girls, having a higher progression rate than boys (p=0.012). Progression to T1D was not affected by being a first degree relative (p=0.925), while children with impaired glucose metabolism at baseline, defined as plasma-glucose 120 min after OGTT ≥7.8 and <11.1 mmol/L, plasma glucose ≥11.1 mmol/L 30, 60 and/or 90 minutes after OGTT and or FPIR <30 in IvGTT, had a higher rate of progression than children with normal glucose tolerance (p=0.013).

Time to clinical diagnosis was not affected by treatment in the full group (p=0.573), or within the stratum groups with 2 or 3-6 autoantibodies (p=0.957 and 0.628 respectively). Moreover, time to diabetes was not significantly affected by treatment within the group of normal and impaired glucose metabolism (p=0.359 and p=0.376, respectively) or by gender (p=0.079 boys, p=0.400 girls, respectively.

Recent investigations have reported that two major groups of children developing islet autoantibodies exist; one that present with IAA as a first autoantibody and this group often include children of low age with HLA genotypes including the DR4-DQ8 haplotype, and the other group often somewhat older children that present with GADA or tGADA first, often of DR3-DQ2-haplotype 2, leading the inventors of the present invention to test the efficacy of GAD-alum versus placebo in these HLA-groups. In this study, including only individuals with high risk to present with clinical T1D, six different HLA-genotypes were represented. Of these the three groups HLA DQ 2/2; DQ 2/X ; and DQ X/X included too few subjects (n=2, 2, 1) to be considered in any analysis. The three remaining HLA-groups included DQ 2/8 (n=25) of which 10 subjects received GAD-alum and 15 received placebo; DQ 8/X (n=11) where six subjects received GAD-alum and 5 placebo; and DQ 8/8 (n=9) where five individuals received GAD-alum and 4 placebo.

Surprisingly, it was found that in the 25 subjects in the DQ2/8 group, two of 10 subjects in the GAD-alum group (20%) presented with T1D within the five-year period, compared to eight of the 15 (53%) individuals that received placebo (table 3) indicating that GAD-alum is effective in delaying T1D in high risk individuals with the DQ2/8 haplotype. Similar findings were not seen in the DQ 8/X nor in the DQ 8/8 groups, where placebo came out better than the GAD-alum treated subjects.

**Table 3**

| **25 DQ2/DQ8 positive individuals** | **# individuals presenting with insulin requiring diabetes in the GAD-alum group (n=10)** | **# individuals presenting with insulin requiring diabetes in the Placebo group (n=15)** |
|---|---|---|
| **Time period (year)** | | |
| 1 | 1 | 0 |
| 2 | 0 | 4 |
| 3 | 0 | 3 |
| 4 | 0 | 1 |
| 5 | 1 | 0 |
| 1-5 | 2 (20%) | 8 (53%) |

### Experiment 3: DiagNode-1

As described by Tavira et al., Journal of Diabetes Research, Volume 2018, Article ID 9391845), an open study was conducted in 12 recent onset T1D patients, where 4µg of GAD formulated in alum was injected directly into the inguinal lymphnode at three times, each with a 30 day period in between injections. Consistent with the subject of this invention it was found that treatment of patients carrying the DR3-DQ2 haplotypes resulted in better HbA1c- and stimulated Area Under the Curve C-peptide data, than patients not carrying the DR3-DQ2 haplotypes.

**Table 4**

| **Patient ID-#** | **A1c at baseline (B)** | **A1c % of B at 15 mths** | **Risk group** | **Haplogenotype** | **Stimulated C- peptide at baseline (AUC-B)** | **AUC % of AUC B at 15 mths** |
|---|---|---|---|---|---|---|
| 1 | 52 | 78,8 | Neutral | DR3-DQ2/DR11-DQ7 | 0,364 | 107,56 |
| 2 | 58 | 93,1 | Increased | DR4/4-DQ8/8 | 0,853 | 79,77 |
| 3 | 66 | 78,8 | Increased | DR4/10-DQ8/5.1 | 0,426 | 82,11 |
| 4 | 68 | 60,3 | Decreased | DR11/13-DQ7v/6.3 | 0,744 | 108,40 |
| 5 | 103 | 43,8 | Increased | DR3/4-DQ2/7 | 0,415 | 85,84 |
| 6 | 78 | 56,4 | Increased | DR3/10-DQ2/5.1 | 0,375 | 71,67 |
| 7 | 41 | 90,2 | High | DR3/4-DQ2/8 | | |
| 8 | 37 | 124,3 | Neutral | DR4/10-DQ7/5.1 | 0,518 | 104,83 |
| 9 | 66 | 60,6 | High | DR3/4-DQ2/8 | 0,518 | 76,57 |
| 10 | 54 | 94,4 | Increased | DR4/4-DQ8/8 | 0,356 | 62,11 |
| 11 | 37 | 105,4 | Increased | DR3/13-DQ2/6.4 | 0,853 | 61,73 |
| 12 | 50 | 98 | Increased | DR4/10-DQ8/5.1 | 0,69 | 54,38 |

**Table 5**

| **DR** | **Group Pat ID** | **A1c % of B at 15 mths** | **AUC % of AUCB at 15 mths** |
|---|---|---|---|
| DR3s-pat #11 | 1+5+6+7+9 | 65,964 | 85,41 |
| DR3s | 1+5+6+7+9+11 | 72,53 | 80,67 |
| DR4s | 2+3+8+10+12 | 97,73 | 76,63 |

## Claims

1. An insulin autoantigen for use in an immunotherapeutic method for treatment or prevention of autoimmune diabetes in an individual, said method comprising
- Determining the HLA haplotype of the individual; and
- Subjecting the individual to a treatment regimen based on said haplotype,
wherein an individual having a an HLA DR4-DQ8 haplotype but not an HLA DR3-DQ2 haplotype is subjected to immunotherapeutic treatment with at least an insulin autoantigen.

2. The insulin autoantigen for use according to claim 1 where the autoimmune diabetes is selected from the group consisting of Type 1 Diabetes Mellitus, 1,5 diabetes, LADA, LADY, SPIDDM, and PIDM.

3. The insulin autoantigen for use according to claim 1, wherein the individual presents with insulin autoantibodies first.

4. The insulin autoantigen for use according to any one of claims 1-3 wherein an administration of insulin autoantigen is oral, sublingual, intramuscular, intradermal, or intra-lymphatic.

5. The insulin autoantigen for use according to claims 1-4 where an insulin antigen is formulated with alum or in saline solution.

## Patentansprüche

1. Insulinautoantigen zur Verwendung in einem immuntherapeutischen Verfahren zum Behandeln oder Verhindern eines Autoimmundiabetes bei einem Individuum, wobei das Verfahren umfasst:
- Bestimmen des HLA-Haplotyps des Individuums; und
- Unterziehen des Individuums einem Behandlungsregime auf der Grundlage des Haplotyps,
wobei ein Individuum mit einem HLA-DR4-DQ8-Haplotyp, aber ohne einen HLA-DR3-DQ2-Haplotyp einer immuntherapeutischen Behandlung mit zumindest einem Insulinautoantigen unterzogen wird.

2. Insulinautoantigen zur Verwendung nach Anspruch 1, wobei der Autoimmundiabetes aus der Gruppe ausgewählt ist, die aus Typ-1-Diabetes mellitus, Typ-1,5-Diabetes, LADA, LADY, SPIDDM und PIDM besteht.

3. Insulinautoantigen zur Verwendung nach Anspruch 1, wobei dem Individuum zuerst Insulinautoantikörper verabreicht werden.

4. Insulinautoantigen zur Verwendung nach einem der Ansprüche 1 bis 3, wobei eine Verabreichung von Insulinautoantigen oral, sublingual, intramuskulär, intradermal oder intralymphatisch ist.

5. Insulinautoantigen zur Verwendung nach den Ansprüchen 1 bis 4, wobei ein Insulinantigen mit Alaun oder in Salzlösung formuliert ist.

## Revendications

1. Auto-antigène d'insuline à utiliser dans un procédé immunothérapeutique pour le traitement ou la prévention du diabète auto-immun chez un individu, ledit procédé comprenant
- la détermination de l'haplotype HLA de l'individu ; et
- la soumission de l'individu à un régime de traitement basé sur ledit haplotype,
dans lequel un individu ayant un haplotype HLA DR4-DQ8 mais pas un haplotype HLA DR3-DQ2 est soumis à un traitement immunothérapeutique avec au moins un auto-antigène d'insuline.

2. Auto-antigène d'insuline à utiliser selon la revendication 1, où le diabète auto-immun est choisi dans le groupe constitué par le diabète sucré de type 1, le diabète 1,5, le LADA, le LADY, le SPIDDM et le PIDM.

3. Auto-antigène d'insuline à utiliser selon la revendication 1, dans lequel l'individu présente d'abord des auto-anticorps d'insuline.

4. Auto-antigène d'insuline à utiliser selon l'une quelconque des revendications 1 à 3, dans lequel l'auto-antigène d'insuline est administré par voie orale, sublinguale, intramusculaire, intradermique ou intra-lymphatique.

5. Auto-antigène d'insuline utilisé selon les revendications 1 à 4, où l'antigène d'insuline est formulé avec de l'alun ou dans une solution saline.
